Europäisches Patentamt

European Patent Office

Office européen des brevets

(19)

(11) Numéro de publication : **0 517 643 A1**

# (12) DEMANDE DE BREVET EUROPEEN

(21) Numéro de dépôt : 92500019.2

(22) Date de dépôt : 03.03.92

(51) Int. Cl.⁵ : **A61M 5/32**

(30) Priorité : **01.06.91 ES 9101329**
**13.02.92 ES 9200306**

(43) Date de publication de la demande :
**09.12.92 Bulletin 92/50**

(84) Etats contractants désignés :
**AT BE CH DE DK FR GB GR IT LI LU MC NL PT
SE**

(71) Demandeur : **Rotlander Ibanez, Adriana
Beatriz
Almagro, 46
E-28010 Madrid (ES)**

(72) Inventeur : **Rotlander Ibanez, Adriana Beatriz
Almagro, 46
E-28010 Madrid (ES)**

(74) Mandataire : **De la Fuente Fernandez, Dionisio
Europatent, S.A. Santa Engracia num. 4-5
E-28010 Madrid (ES)**

(54) **Appareil destructeur d'aiguilles hypodermiques.**

(57)   Appareil destructeur d'aiguilles hypodermiques, formé par une caisse (1) à l'intérieur de
laquelle est installé un dispositif électrique
formé par un transformateur (7) de courant avec
un rectificateur (16) à la sortie et à laquelle est
connectée une plaque flexible (5) susceptible
d'entrer en contact avec l'aiguille (4) à détruire
et avec un disque tournant (12), en employant
du courant continu pourqu'en restant l'aiguille
à une distance adéquate du disque tournant, il
se produise un arc voltaïque qui va fondre
totalement celle-ci. L'orifice d'entrée (3) de l'aiguille se trouve sur une plaque mobile maintenue dans sa position laplus élevée par un
ressort (10) qui en pressionnant quand on introduit l'aiguille permet que celle-ci se rapproche
du disque, qui par son mouvement tournant fait
que les particules détachées soient projetées
vers l'extérieur et soient ramassées à postériori
dans un dépôt dans lequel sont également
ramassés la frette et le goulot de la seringue qui
se cassent dans un deuxième orifice d'entrée
(22) pourvu d'un bord aiguisé coupant.

Fig. 2

EP 0 517 643 A1

Jouve, 18, rue Saint-Denis, 75001 PARIS

## SECTEUR D'APPLICATION DE L'INVENTION

L'appareil destructeur d'aiguilles hypodermiques que l'on décrit s'appliquera dans tous les établissements sanitaires, hôpitaux, cliniques, infirmeries, et autres similaires et dans lesquels on emploie ce type d'aiguilles dans toutes les classes de piqûres, de prises de sang, et d'autres opérations dans lesquelles l'utilisation d'aiguilles est nécessaire.

## ETAT DE LA TECHNIQUE

Il n'exite pas dans l'actualité d'appareils destructeurs d'aiguilles puisqu'on utilise seulement des housses spéciales dans lesquelles on introduit les aiguilles utilisées et ce n'est qu'à postériori qu'elles sont destinées à une destruction massive, ce qui fait que la simple opération d'introduction de l'aiguille utilisée dans sa housse correspondante implique déjà en soi-même un risque pour l'ouvrier de s'égratigner ou de se piquer avec l'aiguille déjà utilisée, et puisse donc ainsi se contagier avec de possibles conséquences graves.

Il existe aussi des seringues spéciales qui empêchent une seconde utilisation de celles-ci moyennant des conformations spèciales qui les autodétruisent après leur usage, mais dans aucun cas on obtient une autodestruction de l'aiguille qui reste intacte dans ces types de seringues autodestructibles.

## DESCRIPTION DE L'INVENTION

L'appareil destructeur d'aiguilles hypodermiques cité est destiné à détruire l'aiguille après son utilisation, en anulant le risque pour la personne qui les manipule de se faire du mal avec celle-ci en esseyant de la garder pour sa destruction postérieure, en basant cette destruction dans l'inutilisation totale de la pointe de l'aiguille par la fonte de celle-ci en la soumettant à l'action d'un courant électrique élevé qui fond la pointe de l'aiguille en granulés par l'action de la chaleur, et qui en plus de laisser la pointe émoussée élimine la possibilité d'un usage postérieur en obturant le canal intérieur de l'aiguille.

Ce courant électrique s'obtient s'obtient au moyen d'une plaue de contact fixe, à laquelle on connecte le pôle d'un générateur, cette plaque au moment de la destruction entre en contact avec l'aiguille métallique à détruire, il y a sous cette plaque une autre plaque tournante à laquelle on connecte le pôle contraire et qui produit, quand la pointe de l'aiguille devient proche, un arc voltaique entre lapointe de l'aiguille et la plaque tournante, qui produit la fusion de lapointe de l'aiguille en la rendant inutilisable et la laissant tout à fait émoussée et son canal intérieur obstruit.

La plaque inférieure tournante permet que les particules dégagées pendant la fusion par l'effet de la force centrifuge soient renvoyées en sens tangentiel pour son ramassage dans un sac spécial que l'on peut extraire facilement de l'appareil pour sa vidange postérieure. Ces petites particules détachées ne risquent pas de contaminer puisque la température élevée atteinte dans la fusion détruit tout type de virus ou de bactéries qui auraient pu exister.

L'alimentation de l'appareil s'obtient moyennant un transformateur de courant alternatif pouvant être branché sur le secteur, et dans le secondaire est inclus un rectificateur pour obtenir du courant continu, en prévoyant en plus que l'alimentation puisse être indifféremment au secteur ou une batterie de 12 V qui permet que l'appareil puisse être utilisé dans des zones où il n'existe pas de distribution régulière d'énergie électrique, dans la campagne, ou dans desinstallations rurales dans lesquelles il n'existe pas de réseau électrique, et dans tous les autres cas similaires qui puissent se présenter avec la simple utilisation d'une batterie de 12 v, comme celle d'une automobile, par exemple.

On introduit dans cet appareil, un dispositif spécial de destruction du goulot de la seringue, n'existant dans aucun des appareils connus, constitué par un orifice aux bords coupants, pour que par le mouvement latéral, le goulot de la seringue soit coupé et qu'il tombe ensuite dans le dépôt pour les résidus que comporte l'appareil.

Par la suite on fera une description détaillée de l'appareil destructeur d'aiguilles hypodermiques présenté en se rapportant aux schémas qui l'accompagnent, dans lesquels on représente uniquement à titre d'exemple non limitatif une forme préférente de réalisation, susceptible de toutes les variations de détail qui ne supposent aucune altération fondamentale des caractéristiques essentielles de celui-ci.

Dans ces schémas on illustre:

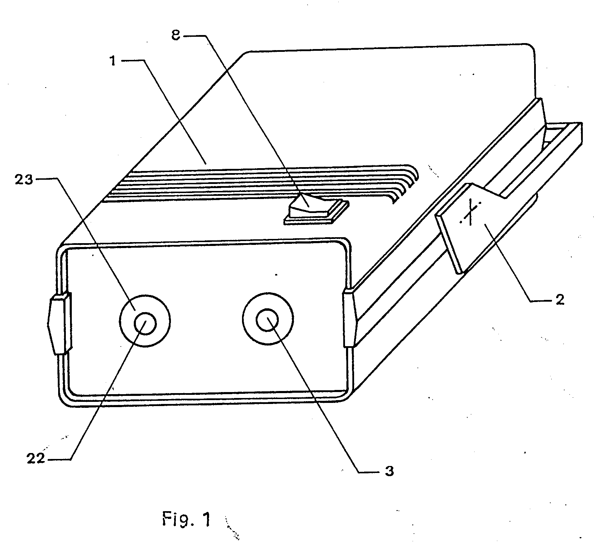

Dans la figure 1: vue générale de la caisse de l'appareil.

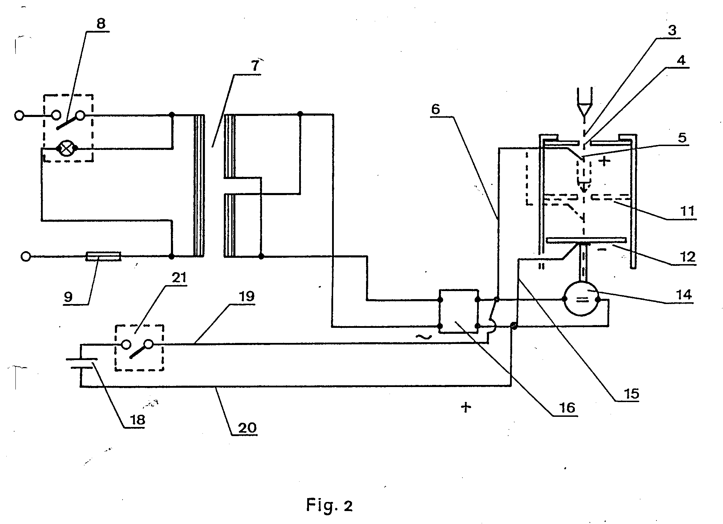

Dans la figure 2: schéma électrique de l'ensemble.

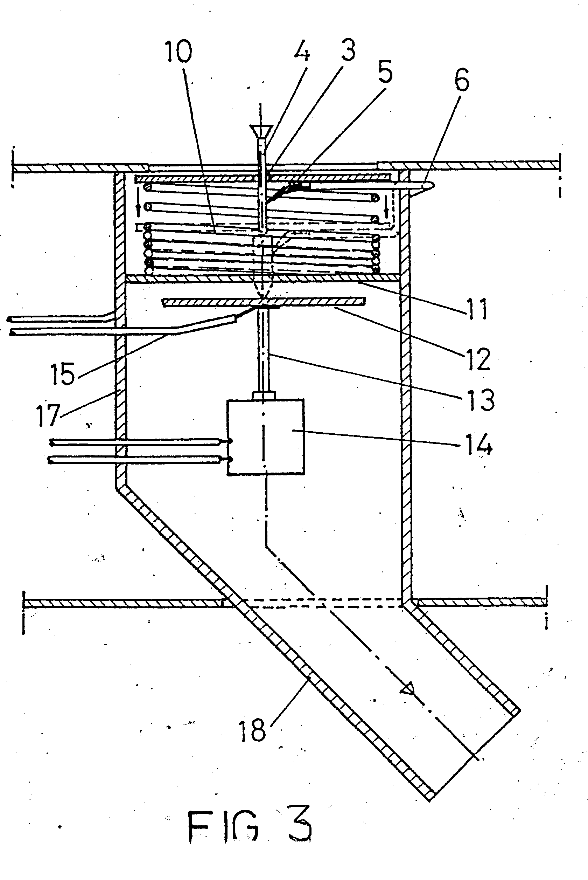

Dans la figure 3: détail schématique, coupe verticale de l'ensemble des éléments mécaniques de l'appareil.

D'après l'exemple d'éxécution représenté, l'appareil destructeur d'aiguilles hypodermiques préconisé est constitué par une caisse (1) avec une anse abattable (2) pour son utilisation et son transport plus commodes, et dans une des faces il existe un orifice (3) pour l'introduction des aiguilles à détruire, et un autre orifice )22) pourvu d'un cercle (23) au bord interne très aiguisé pour la destruction postérieure des goulots des seringues.

A l'intérieur on prévoit le dispositif électrique et mécanique, le premier étant formé par un transformateur (7) dont le primaire reste en connexion avec le réseau par l'intermédiaire d'un interrupteur (8), avec une lampe témoin qui indique que l'appareil est branché, et par un fusible de protection. Ce transformateur obtient la variation de la tension de 220 v en 12 v et 8 a, en prévoyant à la sortie du secondaire du transformateur un diode rectificateur (16) duquel part un pôle vers une plaque flexible (5), par le conducteur (15) avec un disque métallique tournant (12).

Ce même courant continu prévoit le mouvement d'un moteur (14), et à l'intérieur de son axe (13) reste accouplé le dit disque tournant (12).

Sous cet orifice d'entrée (3) pour l'aiguille à détruire, on prévoit une plaque mobile impulsée vers le haut par un ressort (10) qui appuie son extrême contraire sur une plaque fixe (11) avec un orifice central pour le passage de l'aiguille (4), tout cela restant dans une carcasse (17) avec une sortie inférieure (18) vers un dépôt pour les résidus accessible depuis l'extérieur au moyen d'un couvercle existant dans la caisse (1).

L'appareil ainsi organisé, une fois mis en marche, le moteur (14) met en mouvement le disque (12), qui reste en contact avec le pôle (15), et en introduisant l'aiguille (4) celle-ci entre en contact avec la lame flexible (5) accouplée au pôle contraire (6), et en appuyant vers le bas l'effet du ressort fait que la pointe de l'aiguille se rapproche du disque tournant (12), jusqu'à arriver à une distance à laquelle il se produit un arc voltaique entre la pointe et le disque qui détruit celle-ci par fusion, et ensuite toute la longueur de l'aiguille, au fur et à mesure qu'elle descend jusqu'à la frette finale.

Le mouvement tournant du disque (12) fait aussi que les particules dégagées durant la fusion soient lancées vers les parois de la carcasse (17) par l'effet de centrifugation correspondant, ce qui fait impossible que les dépôts provenant de la fusion restent sur le disque(12), et donc le fonctionnement est toujours assuré, l'arc se produisant constamment , puisque l'aiguille et le disque n'arrivent pas à être totalement en contact.

Le fonctionnement est toujours assuré, que ce soit en le branchant au secteur ou avec la batterie, on obtient entre la plaque flexible et le disque tournant un courant de 12 V et 8 A, suffisant pour obtenir l'arc voltaique qui doit détruire l'aiguille par la fusion totale de celle-ci.

La destruction de l'aiguille finie, on procède à introduire la frette de celle-ci avec le goulot de la seringue dans l'autre orifice (22), en faisant un léger mouvement latéral, qui par l'effet du bord aiguisé du cercle (23), fair que le dit goulot soit coupé, les restes tombant à l'intérieur de la caisse, dans le même dépôt oû sont rammassés lesrésidus de la fusion.

Ce dépôt pourra être vidé autant de fois que précis, sans aucun danger de contamination, car les particules après avoir été soumises à une très haute température ne possèdent aucun type de virus ou de bactéries dangereux, mais cependant ce dépôt peut être détruit ou substitué sans être ouvert, pour une sécurité maximum.

La forme, les matériaux et les dimensions pourront varier et en général tout ce qui soit accessoire et secondaire, et qui n'altère, ne change ou ne modifie jamais l'essentialité de l'appareil décrit.

**Revendications**

1. Appareil destructeur d'aiguilles hypodermiques se caractérise parcequ'il est constitué par une caisse dans laquelle est inclus un orifice d'entrée pour l'aiguille à détruire et sous lequel il y a une plaque mobile appuyée sur un ressort, qui la maintient dans une position plus haute, et sous ce ressort une autre plaque fixe sous laquelle il y a un disque rotatif gràce à un moteur électrique, et on prévoit dans la zone d'entrée de l'aiguille une plaque en connexion à un pôle transformateur, et on prévoit aussi que le disque rotatif soit connecté au pôle contraire pour que la pointe de l'aiguille se rapprochant du disque, il se produise entre l'aiguille et le disque, par pression et compression du ressort un arc voltaique qui provoque la fusion de l'aiguille et la détruit.

2. Appareil destructeur d'aiguilles hypodermiques, d'après revendication première, se caractérise parceque le dispositif de destruction est inclus dans un cylindre dont les parois intérieures ramassent les particules incandescentes qui se dégagent de la destruction par l'effet centrifuge du disque rotatif, en prévoyant un conduit intérieur accoudé pour leur dépôt dans un réceptacle adéquat.

3. Appareil destructeur d'aiguilles hypodermiques, d'après revendication première et deuxième, se caractérise parcequ'il inclue une prise de courant, un interrupteur, des fusibles de protection, et un transformateur de puissance et ampérage adéquat pour obtenir l'intensité élevée nécessaire pour la production de l'arc voltaique qui origine la fusion, en prévoyant dans le secondaire du transformateur, l'installation d'un diode rectificateur pour l'emploi de courant continu autant dans le moteur que dans l'arc voltaique.

4. Appareil destructeur d'aiguilles hypodermiques, d'après les revendications première à troisième, se caractérise parcequ'il prévoit parallèlement au dispositif électrique un transformateur et une prise de courant au secteur, un autre dispositif alimenté par une batterie de 12 V, qui alimente directement le moteur et le système de l'arc, avec un interrupteur qui permet d'utiliser l'une ou l'autre indifféremment en fonction des besoins de chaque instant.

5. Appareil destructeur d'aiguilles hypodermiques, d'après les revendications première à quatrième, se caractérise parcequ'on a prévu à l'extérieur de la caisse qui contient le dispositif à côté de l'orifice d'entrée pour l'aiguille à détruire, un ou plusieurs orifices, à l'entrée desquels on adapte une plaque sous forme de couronne circulaire avec un rebord très aiguisé dans sa circonférence intérieure pour l'introduction et le sectionnement du goulot de la seringue, en la rendant inutilisable.

Tel qu'il est décrit et revendiqué dans la présente mémoire descriptive formée par huit feuilles dactylographiées au recto et de trois schémas qui l'illustrent.

Fig. 1

Fig. 2

FIG 3

EP 0 517 643 A1

**Office européen des brevets**

# RAPPORT DE RECHERCHE EUROPEENNE

Numero de la demande

EP     92 50 0019

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 332 584 (JEABOR S.A.) <br> * revendications; figures * <br> --- | 1-5 | A61M5/32 |
| A | EP-A-0 136 392 (CH'ING-LUNG) <br> * revendications; figures * <br> --- | 1-5 | |
| A | DE-U-9 004 788 (DUDERSTAEDTER DENTAL-LABOR) <br> * revendications; figures * <br> --- | 1-5 | |
| A | US-A-4 971 261 (SOLOMONS) <br> * abrégé; figures * <br> ----- | 1-5 | |

**DOMAINES TECHNIQUES RECHERCHES (Int. Cl.5)**

A61M

Le présent rapport a été établi pour toutes les revendications

| Lieu de la recherche | Date d'achèvement de la recherche | Examinateur |
|---|---|---|
| LA HAYE | 03 SEPTEMBRE 1992 | MIR Y GUILLEN V. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

8